(19) Europäisches Patentamt / European Patent Office / Office européen des brevets

(11) EP 4 072 609 B1

(12) EUROPEAN PATENT SPECIFICATION

(45) Date of publication and mention of the grant of the patent:
03.06.2026 Bulletin 2026/23

(21) Application number: 20829272.2

(22) Date of filing: 11.12.2020

(51) International Patent Classification (IPC):
A61L 27/38 (2006.01)    A61L 27/48 (2006.01)
A61L 27/34 (2006.01)

(52) Cooperative Patent Classification (CPC):
(C-Sets available)
A61L 27/3808; A61L 27/34; A61L 27/3813;
A61L 27/3834; A61L 27/48; A61L 2430/16   (Cont.)

(86) International application number:
PCT/US2020/064561

(87) International publication number:
WO 2021/119460 (17.06.2021 Gazette 2021/24)

(54) **REINFORCED BIOPOLYMERS**

VERSTÄRKTE BIOPOLYMERE

BIOPOLYMÈRES RENFORCÉS

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 13.12.2019 US 201962947933 P

(43) Date of publication of application:
19.10.2022 Bulletin 2022/42

(73) Proprietor: W. L. Gore & Associates, Inc.
Newark, DE 19711 (US)

(72) Inventors:
• BALAJI, Gopalan V.
Newark, Delaware 19711 (US)
• PARSONS, Bernadette
Newark, Delaware 19711 (US)

(74) Representative: HGF
HGF Limited
4th Floor, 1 City Square
Leeds LS1 2ES (GB)

(56) References cited:
WO-A1-2013/040559    WO-A2-2008/127653

• STODOLAK E. ET AL: "A composite material used as a membrane for ophthalmology applications", COMPOSITES SCIENCE AND TECHNOLOGY, vol. 70, no. 13, 1 November 2010 (2010-11-01), AMSTERDAM, NL, pages 1915 - 1919, XP055785377, ISSN: 0266-3538, Retrieved from the Internet <URL:https://core.ac.uk/download/pdf/208444601.pdf> DOI: 10.1016/j.compscitech.2010.07.007
• KEARNS VICTORIA R ET AL: "The formation of a functional retinal pigment epithelium occurs on porous polytetrafluoroethylene substrates independently of the surface chemistry", JOURNAL OF MATERIALS SCIENCE: MATERIALS IN MEDICINE, SPRINGER NEW YORK LLC, UNITED STATES, vol. 28, no. 8, 13 July 2017 (2017-07-13), pages 1 - 14, XP036291571, ISSN: 0957-4530, [retrieved on 20170713], DOI: 10.1007/S10856-017-5926-3
• LEGEAIS J M ET AL: "Expanded fluorocarbon for keratoprosthesis cellular ingrowth and transparency", EXP. EYE RES,, vol. 58, 1 January 1994 (1994-01-01), pages 41 - 52, XP008116446, DOI: 10.1006/EXER.1994.1193

EP 4 072 609 B1

• TONSOMBOON K ET AL: "Gelatin nanofiber-reinforced alginate gel scaffolds for corneal tissue engineering", ENGINEERING IN MEDICINE AND BIOLOGY SOCIETY (EMBC), 2013 34TH ANNUAL INTERNATIONAL CONFERENCE OF THE IEEE, IEEE, 3 July 2013 (2013-07-03), pages 6671 - 6674, XP032489063, ISSN: 1557-170X, [retrieved on 20130925], DOI: 10.1109/EMBC.2013.6611086

(52) Cooperative Patent Classification (CPC): (Cont.)

C-Sets
**A61L 27/34, C08L 89/06;
A61L 27/48, C08L 27/18**

**Description**

BACKGROUND

Cell transplantation holds significant potential for regenerating and repairing tissues and treating disease and disorders. Cells transplanted in suspension at or near their intended target may adhere and integrate into the target tissue. However, many factors can affect their rate of incorporation into the target tissue, and the cells may not effectively adhere to the intended target. Biopolymer scaffolds can provide a means for retaining cells at their intended target, increasing the rate of incorporation of the transplanted cells into the target tissue.

*In vitro* cell culture on polymer scaffolds provides a strategy for tissue creation and cell transplantation. A specific cell line is seeded onto the polymer scaffold. The polymer scaffold should provide good biocompatibility, controllable biodegradability, appropriate tensile strength, and flexibility. While collagen has often been used as a biopolymer scaffold, extensive cross-linking of the collagen is often required to provide the biopolymer with the requisite tensile strength which in turn reduces its flexibility, making it difficult to manipulate during both cell culture and transplantation. Furthermore, such biopolymer scaffolds are generally hazy and unsuitable for implantation in the eye.

WO 2013/040559 A1 discloses a scaffold comprising a biopolymer, preferably gelatin, and a synthetic polymer selected from the group of (meth)acrylate-oligolactide-PEO-oligolactide. PEO-PPO-PEO copolymers (Pluronics), poly(phosphazene), poly(methacrylates), poly(N-vinylpyrrolidone) PL(G)A-PEO-PL(G)A copolymers, poly(ethylene imine), and poly(ethylene glycol) diacrylate. The scaffold is 95% transparent with respect to visible light, and is porous to vital nutrients. The surface of the scaffold can be modified with fibronectin, laminin, vitronectin, or RGD, which can promote cell adhesion and proliferation. The scaffold is used in corneal endothelium transplantation, and it may be used as an implantable cell therapy device when the scaffold is seeded with a monolayer of cells, such as corneal endothelial cell layers.

SUMMARY

While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.

FIG. 1 is an illustration of a cross-sectional view of a reinforced biopolymer in accordance with an embodiment.
FIG. 2 is an illustration of a cross-sectional view of a reinforced biopolymer in accordance with an embodiment.
FIG. 3 is an illustration of a cross-sectional view of a reinforced biopolymer in accordance with an embodiment.

DETAILED DESCRIPTION

Definitions and Terminology

This disclosure is not meant to be read in a restrictive manner. For example, the terminology used in the application should be read broadly in the context of the meaning those in the field would attribute such terminology.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

Description of Various Embodiments

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

Various aspects of the present disclosure are directed toward reinforced biopolymers. The reinforced biopolymers may be configured to support and maintain cells on a surface thereof. The reinforced biopolymer may, for example, be used to support a monolayer of cells on a surface of the reinforced biopolymer. The cells may be, for example, stem cells, epithelial cells, endothelial cells, or retinal pigment epithelial cells. The reinforced biopolymers, as discussed in detail below, allow for mass transport across the reinforced biopolymers, have a high optical transparency, and have a sufficient toughness to endure manipulation, including bending and/or folding.

The reinforced biopolymers described herein may be used as a device or in a device, in methods, and in systems and may be configured to deliver a monolayer of cells to a target site. In certain instances, and as discussed in further detail below, the mechanical properties of the reinforced biopolymers are designed to avoid tearing or ripping during implantation and once implanted in vivo.

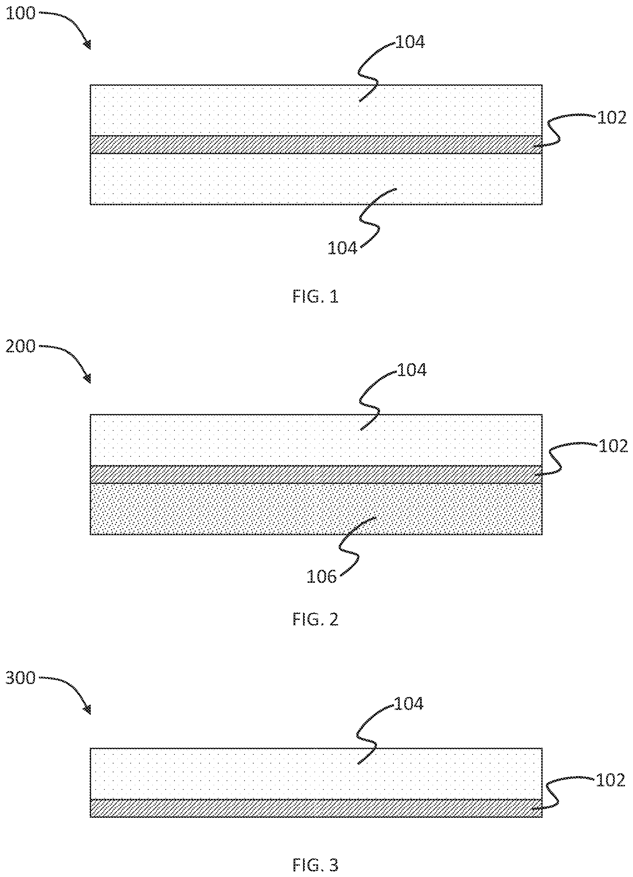

FIG. 1 is an illustration of an example reinforced biopolymer 100 in accordance with an embodiment. In certain embodiments, the reinforced biopolymer 100 has a composite structure including a synthetic support membrane 102 and biopolymer 104. As illustrated, a layer of biopolymer 104 occurs on each side of the synthetic support membrane 102, with the synthetic support membrane 102 positioned approximately centrally between the two layers of biopolymer 104.

FIG. 2 is an illustration of an example reinforced biopolymer 200 in accordance with another embodiment. Reinforced biopolymer 200 includes a synthetic support membrane 102, a first layer of biopolymer 104, and a second layer of biopolymer 106, wherein the first layer of biopolymer 104 and the second layer of biopolymer 106 are different biopolymers or different formulations of the same biopolymer. As illustrated, the synthetic support membrane 102 is positioned approximately centrally between the first layer of biopolymer 104 and the second layer of biopolymer 106.

Although the synthetic support membrane 102 is positioned approximately centrally between two layers of biopolymer in each of reinforced biopolymer 100 of FIG. 1 and reinforced biopolymer 200 of FIG. 2, it will be understood that the synthetic support membrane 102 does not need to be so positioned. In some embodiments, for example, a biopolymer layer occurring on a first side of synthetic support membrane 102 can have a greater thickness than the biopolymer layer occurring on a second side of the synthetic support membrane 102. For example, the thickness of the biopolymer on the first side of the synthetic support membrane 102 relative to the thickness of the biopolymer on the second side of the synthetic support membrane 102 can be expressed as a ratio of about 1:1 (i.e., synthetic support membrane 102 is positioned approximately centrally), about 2:1, about 3:1, about 3:2, about 4:1, about 4:3, about 5:1, about 5:2, about 5:3, about 5:4, about 6:1, about 6:5, about 7:1, about 7:2, about 7:3, about 7:4, about 7:5, about 7:6, about 8:1, about 8:3, about 8:5, about 8:7, about 9:1, about 9:2, about 9:4, about 9:5, about 9:7, about 10:1, about 10:3, about 10:7, or about 10:9. Other ratios are also contemplated.

FIG. 3 is an illustration of an example reinforced biopolymer 300 in accordance with another embodiment. Reinforced biopolymer 300 includes a synthetic support membrane 102 and a layer of biopolymer 104 occurring only on a first side of the synthetic support membrane 102. Because biopolymer 104 may be imbibed into synthetic support membrane 102, a nominal amount of biopolymer 104 may extend to a second side of the synthetic support membrane 102.

In certain embodiments, the synthetic support membrane 102 is biologically inert. The synthetic support membrane 102 includes at least one of expanded polytetrafluoroethylene (ePTFE) and expanded polyethylene. In some embodiments, the synthetic support membrane 102 includes ePTFE.

The ePTFE synthetic support membrane of some embodiments is biaxially oriented and has high intrinsic strength, and can be prepared according to the general methodology described in U.S. Patent 3,953,566 to Gore The biaxially oriented ePTFE synthetic support membrane is highly crystalline (i.e., a crystallinity index of at least 94%) and has a matrix tensile strength in both the longitudinal and transverse directions of at least 600 MPa. The ePTFE synthetic support membrane may be comprised of a plurality of stacked ePTFE layers, wherein each layer has an areal density of less than 100 $mg/m^2$.

In some instances, the ePTFE synthetic support membrane is prepared according to the methodology describe in U.S. Patent 7,306,729 to Gore. In other instances, the ePTFE synthetic support membrane is prepared according to the methodology describe in U.S. Pub. No. 2012/0065649. In other instances, the ePTFE synthetic support membrane is prepared according to the methodology described in U.S. Patent 5,814,405 to Gore.

In some embodiments, the synthetic support membrane is configured to permit mass transport across the synthetic support membrane when included in a reinforced biopolymer. By permitting mass transport across the synthetic support membrane, nutrients can diffuse across the membrane to reach cells on the surface of the biopolymer, and cellular waste byproducts can diffuse away from the cells and across the membrane. The mass transfer coefficient of the synthetic support membrane can be adapted to a desired use, increasing or decreasing mass transport potential across the synthetic support membrane. The mass transfer coefficient can be controlled by adjusting membrane pore size and/or membrane layering.

The synthetic support membrane of the reinforced biopolymer has a thickness of about 0.5 $\mu$m to about 10 $\mu$m. In certain embodiments, the synthetic support membrane has a thickness of about 0.5 $\mu$m, about 1 $\mu$m, about 1.5 $\mu$m, about 2 $\mu$m, about 2.5 $\mu$m, about 3 $\mu$m, about 3.5 $\mu$m, about 4 $\mu$m, about 4.5 $\mu$m, about 5 $\mu$m, about 5.5 $\mu$m, about 6 $\mu$m, about 6.5 $\mu$m, about 7 $\mu$m, about 7.5 $\mu$m, about 8 $\mu$m, about 8.5 $\mu$m, about 9 $\mu$m, about 9.5 $\mu$m, or about 10 $\mu$m. As described in detail below, in certain instances, the wetting out process can cause the synthetic support membrane to "collapse," with a decrease in membrane thickness. The thicknesses provided above refer to the "collapsed" synthetic support membrane following wetting out and as it appears in the composite reinforced biopolymer.

In certain embodiments, the biopolymer includes collagen, gelatin, laminin, fibronectin, fibrinogen, elastin, thrombospondin, heparan sulfate, chondroitin sulfate, polysaccharides, alginate, chitosan, glycosaminoglycan, hyaluronic acid, or a combination of any two or more of these. In particular embodiments, the biopolymer includes a collagen. Many of these components may exist in different forms or as different types. For example, there are many different types of collagen (e.g., Types I, II, III, etc.) and gelatin (e.g., Type A, Type B). Further, these components may be derived from a variety of sources. It is contemplated that any type or form, derived from any source, may be included in a biopolymer described herein.

In some embodiments, the biopolymer also includes one or more additives, such as, for example, a coating or imbibing aid, a viscosity modifier, a density modifier, or a biopolymer cross-linker.

A single biopolymer layer occurring on one side of the synthetic support membrane has a thickness of about 0 $\mu$m to about 95 $\mu$m. In some embodiments, the biopolymer is fully imbibed into the synthetic support membrane on one side of the synthetic support, the biopolymer layer thus having a thickness of about 0 $\mu$m.

In certain instances, the biopolymer of the reinforced biopolymer is cross-linked. Cross-linking the biopolymer can increase its tensile strength and thermostability. Methods for cross-linking biopolymer elements contemplated herein such as collagen, gelatin, laminin, fibronectin and the like are known in the art. Cross-linking, while increasing tensile strength, can have the negative effect of reducing the 'stickiness' of the biopolymer and it ability to retain cells and/or adhere to tissue *in vivo.* An advantage of the reinforced biopolymers disclosed herein is that because of the synthetic support membrane, the biopolymer does not need to be crosslinked to increase its tensile strength; the strength and toughness of the reinforced biopolymer is provided by the synthetic support membrane. Although the biopolymer may still be cross-linked, it does not need to be cross-linked as extensively as it would need to be if the synthetic support membrane were not present. This permits the biopolymer to retain its stickiness and ability to retain cells and/or adhere to tissues *in vivo.* Cross-linking the biopolymer may be done to, for example, increase the thermostability of the biopolymer so that it may be implanted *in vivo* without liquefying. The extent or level of cross-linking necessary to achieve the desired thermostability is generally lower than that required to achieve significant gains in tensile strength. Where the reinforced biopolymer includes a biopolymer layer on both sides of the synthetic support membrane, as in reinforced biopolymers 100 and 200 depicted by FIGs. 1 and 2 respectively, the surfaces of the two biopolymer layers can be similarly cross-linked, or differentially cross-linked to provide each surface with distinct surface properties (e.g., differing levels of cell adhesion, or stickiness to biological tissues).

In certain embodiments, a surface of a biopolymer layer is configured to support cellular growth, cellular adhesion, or both cellular growth and adhesion. In certain embodiments, a layer of cells are grown directly on the biopolymer layer's surface. In other embodiments, a suspension of cells is seeded onto the biopolymer layer's surface and the cells allowed to adhere to the biopolymer layer's surface. In certain instances, the cells grown on the biopolymer layer's surface or the cells adhered to the biopolymer layer's surface form a cellular monolayer (i.e., a layer of cells that is one-cell-thick).

Where the reinforced biopolymer includes a biopolymer layer on both sides of the synthetic support membrane, as in reinforced biopolymers 100 and 200 depicted by FIGs. 1 and 2 respectively, the surface distal to the synthetic support membrane of either biopolymer layer may be configured to support cellular growth, cellular adhesion, or both cellular growth and cellular adhesion. In certain instances, the surface distal to the synthetic support membrane of one biopolymer layer can be configured to support cellular growth, cellular adhesion, or both cellular growth and cellular adhesion and the surface distal to the synthetic support of the other biopolymer layer can be configured to promote adherence to a biological tissue, or to prevent adherence and preclude binding of proteins and/or cells to the surface.

Where the reinforced biopolymer includes a biopolymer layer only on one side of the synthetic support membrane, as in reinforced biopolymer 300 depicted by FIG. 3, the surface distal to the synthetic support membrane of the single biopolymer layer may be configured to support cellular growth, cellular adhesion, or both cellular growth and cellular adhesion. In certain instances, the surface distal to the synthetic support of one biopolymer layer can be configured to support cellular growth, cellular adhesion, or both cellular growth and cellular adhesion and the surface of the synthetic support membrane opposite of the single biopolymer layer can be configured to promote adherence to a biological tissue, or to prevent adherence and preclude binding of proteins and/or cells to the surface.

The biopolymer can be selected to support growth of a desired cell type. In some embodiments, it may be desirable to grow corneal endothelial cells, corneal epithelial cells, retinal pigment epithelial cells, photoreceptor cells, Müller glial cells, ganglion cells, endothelial cells, epithelial cells, pericytes, or a combination thereof on a surface of the reinforced biopolymer. In certain instances, these cells form a monolayer of cells on a surface of the reinforced biopolymer. Biopolymer substrates suitable for supporting growth of these and other cell types are known in the art.

In some embodiments, the surface of the biopolymer layer configured to support cellular growth, cellular adhesion, or both cellular growth and cellular adhesion is functionalized. The surface may be functionalized by, for example, including cell adhesion molecules, antigens, epitopes, and/or stem cell differentiation factors at the surface of the biopolymer layer. It will be recognized that such functionalization factors are cell-specific, and can be selected according to the specific cell type.

In certain instances, the biopolymer layer(s) is configured to minimize or avoid an inflammatory response when implanted *in vivo.*

The thickness of the reinforced biopolymer can be from about 0.5 $\mu$m to about 100 $\mu$m. In some embodiments, the reinforced biopolymer can have a thickness of about 10 $\mu$m to about 50 $\mu$m. In other embodiments, the reinforced biopolymer can have a thickness of about 20 $\mu$m to about 30 $\mu$m. In a particular embodiment, the reinforced biopolymer can have a thickness of about 20 $\mu$m.

The reinforced biopolymer described herein has a toughness of at least about 30 KJ/m$^3$. As the toughness of the synthetic support membrane is a few orders of magnitude greater than that of the biopolymer layer(s) - even if crosslinked, the toughness of the synthetic support membrane approximates the toughness of the reinforced biopolymer. The contribution of the biopolymer to the toughness of the reinforced biopolymer can be neglected.

The synthetic support membrane-biopolymer composite construction of the reinforced biopolymers described herein results in the biopolymer having sufficient tensile strength and toughness to withstand handling and manipulation during both cell culture procedures and *in vivo* implantation. Unlike a substrate made up only of biopolymer, which requires extensive cross-linking to achieve sufficient tensile strength to withstand manipulation and implantation, the described reinforced biopolymers are flexible and tough, while minimizing the need to further manipulate (e.g., cross-link) the biopolymer layer(s).

The reinforced biopolymer described herein has a measured optical transparency of at least about 85%. In some embodiments, a reinforced biopolymer described herein has a measured optical transparency of at least about 90%. Optical transparency is measured in air with the biopolymer in a fully hydrated state. When measured in air, Fresnel reflection at the outside interfaces between the sample and its environment reduces transmission values compared to optical transparency of the reinforced biopolymer when in use in accordance to the present disclosure. It will be understood that the optical transparency of the reinforced biopolymer in use will be higher than the measured value in air.

Optically transparent reinforced biopolymers can be used, for example, as substrates or vehicles for delivering cells to the eye without adversely affecting the eye's optics. For example, a monolayer of corneal endothelial cells grown on a surface of a biopolymer layer of a reinforced biopolymer can be implanted at the posterior surface of the cornea, providing a layer of healthy endothelial cells to the cornea (i.e., an endothelial keratoplasty). In another example, retinal pigment epithelial cells can be introduced directly to the retina as a monolayer formed on a surface of a reinforced biopolymer. In both of these examples, the reinforced biopolymers allow normal optical function of the eye, with good transparency and minimal scattering.

The transparency of ePTFE is largely governed by how much residual scattering is present in the bulk of the material. To obtain high specular (clear image-forming) transmission, diffuse (scattered) components in both transmission and reflection must be minimized. This is typically done by either thinning of the structure or incorporation of a substance with a near-matching refractive index (around 1.37 for ePTFE) into ePTFE's microstructure. In some embodiments, the ePTFE synthetic support membrane is prepared according to the methodology describe in U.S. Patent 7,306,729 to Gore, which is hereby incorporated by reference in its entirety. Some members of this family of membranes possess high clarity while having high degrees of porosity, which normally reduces the amount of visible light passing through the membrane. Certain membranes can have a light transmission, or optical transparency, of at least 50% and a porosity of at least 50%, while light transmission values of 85% and higher are obtainable in membranes that are greater than 75% porous.

The amount of air trapped within the synthetic support membrane and the hydration index of imbibed substances are important determinants in the optical transparency of the support membrane, contributing to the optical transparency of the reinforced biopolymer. In some embodiments, all or substantially all air is expelled from the synthetic support membrane during a wetting out process that occurs prior to the synthetic support membrane being imbibed with a biopolymer. In some instances, the wetting out process results in the membrane "collapsing" on itself. For example, an ePTFE membrane having a thickness of about 5 $\mu$m prior to wetting out may collapse to a thickness of about 1 $\mu$m. The wetted out synthetic support membrane is then prepared and imbibed with the desired biopolymer to produce a reinforced biopolymer that is free or substantially free of air. In some instances, where the reinforced biopolymer is imbibed with biopolymer and is free or substantially free of air, it is considered to be "fully imbibed."

In certain embodiments, the biopolymer has a refractive index that is the same as or substantially similar to the refractive index of the synthetic supporting membrane.

In another aspect, provided herein are implantable cell therapy devices. The implantable cell therapy device includes a reinforced biopolymer and a monolayer of cells on a surface of the biopolymer that is distal to the synthetic support membrane. The monolayer of cells can include, but is not limited to, corneal endothelial cells, corneal epithelial cells, retinal pigment epithelial cells, photoreceptor cells, Müller glial cells, ganglion cells, endothelial cells, epithelial cells, pericytes, stem cells, and combinations thereof. In some embodiments, the stem cells are pluripotent stem cells. In other embodiments, the stem cells are multipotent stem cells.

Methods of treating a disease or condition are discussed. In some instances, the disease or condition affects a cellular layer in a subject. The disease or condition can be, for example, corneal endothelial dystrophy, a disease or condition affecting the retinal pigment epithelium, or a disease or condition affecting the photoreceptors. The methods generally include surgically implanting an implantable cell therapy device described herein on or near the diseased site or otherwise affected area, where the implantable cell therapy device includes a monolayer of cells selected to treat or otherwise improve the disease or condition. For example, corneal endothelial dystrophy can be treated by surgically implanting a cell therapy device including a monolayer of corneal endothelial cells at a posterior surface of the cornea. In some embodiments, the implantation procedure includes first removing some or all of the diseased or non-functional corneal endothelium. A disease or condition of or affecting retinal pigment epithelium can be treated by surgically implanting a cell therapy device including a monolayer of retinal pigment epithelial cells at the retina. A disease or condition of or affecting photoreceptors can be treated by surgically implanting a cell therapy device including a monolayer of photoreceptor cells at the retina.

TEST METHODS

It should be understood that although certain methods and equipment are described below, other methods or equipment determined suitable by one of ordinary skill in the art may be alternatively utilized.

Test Methods

*Thickness Measurements*

Synthetic support membrane thickness is measured via a non-contact measurement using a Keyence LS-7010.

Reinforced biopolymer thickness and biopolymer layer thickness (in both hydrated and dehydrated conditions) is determined by laser scanning confocal microscopy (LSCM) cross-sectioning. The reinforced biopolymer can be stained (e.g., with Rhodamine) to offset the biopolymer layer from the synthetic support membrane. Thickness of the synthetic support membrane, whether alone or when incorporated into the reinforced biopolymer, can also be determined by LSCM. LSCM is used as the composite thickness (and layers thereof) is measured in a fully hydrated state. The thickness of the reinforced biopolymer in a partially hydrated or dry state is significantly thinner (e.g., about 200%-500%).

*Tensile Strength & Toughness*

Tensile properties of the synthetic support membrane, which approximate the tensile properties of the reinforced biopolymer, are determined in accordance with ASTM D412-Dogbone F. Peak tensile load, tensile load, and toughness (i.e., area under the curve up to peak tensile load) can each be calculated from the standard test data.

*Optical Transparency*

The spectral transmission and haze of a specimen (membrane, film, etc.) is measured in air using a Shimadzu UV-2700 spectrophotometer. The instrument contains an integrating sphere and has a range of measurement from 185 to 900nm. Both haze and transmission measurements are described in ASTM standard D1003-13 and implemented accordingly.

EXAMPLE

A 10% gelatin solution was prepared with Type A gelatin. A 3 mil (76.2 $\mu$m thick) ePTFE membrane was placed and fixated onto a table. The membrane was then hydrophilically treated and dried. The dry, hydrophilically treated membrane was then mounted onto a hoop. An excess of the gelatin solution was pipetted onto the membrane, and a drawdown procedure using RDS15 Mayer bar was carried out to form a layer of gelatin on the membrane. This procedure involved putting a line of gelatin solution (in excess) across a top edge of the membrane and drawing it down with the Mayer bar. Care was taken not to move the membrane. The membrane immediately wetted out with the gelatin. The formed reinforced biopolymer composite was then allowed to dry. The composite was optionally rewetted to allow for easier peel off. Gelatin layer thickness can be controlled by using different drawdown bars. The membrane with gelatin layer was dried and hydrophilically treated.

Gelatin of the gelatin layer was crosslinked using a 1-ethyl-3-(3-dimethylaminopropyl) carbodiimide-N-hydroxysuccinimide (EDC-NHS) crosslinking procedure (see, e.g., Kuijpers et al. (2000), Journal of Biomaterials Science, Polymer Edition, 11(3):225-243). Concentrations of EDC/NHS were altered to generate cross linking from 5% to '100%' (functional groups crosslinked to their maximum in view of steric hindrance). The gelatin weight of the reinforced biopolymer was determined, and the amount of gelatin was used to calculated how much EDC would be required to activate carboxylic acid residues. A 2.5% w/v EDC solution with NHS (molar ratio NHS/EDC = 0.2) was prepared. MES buffer (0.05M, pH 5.3) was used to immerse the ePTFE-gelatin composite so the concentration of gelatin was 1 g/ 50 ml. The required quantity of EDC/NHS solution was subtracted from this volume, and the required quantity of EDC/NHS solution was added. The following formula illustrates the calculation:

$$g\ gelatin * \frac{79 mmol\ COO^{-*}}{100g\ gelatin} * \frac{x mmol\ EDC}{1 mmol\ COO^{-}} * \frac{1 mol\ EDC}{1000 mmol\ EDC} * \frac{191.70g}{1 mol\ EDC} = g\ EDC$$

where x is the degree of carboxylic acid residue activation desired. Note that 79mml COO- per 100g gelatin is only true for gelatin type A, based on an average of 78-80mmol.

$$\frac{g\ EDC}{2.5\ or\ 5\%\ w/v} = mL\ EDC\ solution$$

Reinforced biopolymers were incubated in the refrigerator for 16 hours before quenching in quenching solution for 2 hours. The reinforced biopolymers were washed in deionized water for 4 hours, including replacement of solution and agitation to assure removal of reaction byproducts.

The reinforced biopolymers were then mounted on a PTFE ring, which was placed in a 24 well plate. The reinforce biopolymers were kept hydrated until cell culture was seeded onto the samples.

The invention of this application has been described above both generically and with regard to specific embodiments. It will be apparent to those skilled in the art that various modifications and variations can be made in the embodiments without departing from the scope of the disclosure. Thus, it is intended that the embodiments cover the modifications and variations of this invention provided they come within the scope of the appended claims.

## Claims

1. A reinforced biopolymer (100, 200, 300) comprising i) a synthetic support membrane (102) including at least one of: expanded polytetrafluoroethylene (ePTFE); and expanded polyethylene and ii) a biopolymer (104), the reinforced biopolymer having

   a. a measured optical transparency of at least 85%;
   b. a thickness of 100 $\mu$m or less; and
   c. a toughness of at least 30 KJ/m$^3$ ,

   wherein the synthetic support membrane has a thickness of 0.5 $\mu$m to 10 $\mu$m; and wherein a single biopolymer layer occurring on one side of the synthetic support membrane has a thickness of 0 $\mu$m to 95 $\mu$m, wherein the optical transparency, the thickness and the toughness are measured by the methods defined in the description.

2. The reinforced biopolymer of claim 1, wherein the biopolymer includes a material selected from the group consisting of collagen, gelatin, laminin, fibronectin, fibrinogen, elastin, thrombospondin, heparan sulfate, chondroitin sulfate, polysaccharides, alginate, chitosan, glycosaminoglycan, hyaluronic acid, and combinations thereof.

3. The reinforced biopolymer of claim 1 or claim 2, wherein the biopolymer is functionalized to support cellular growth, cellular adhesion, or both cellular growth and adhesion.

4. The reinforced biopolymer of any one of claims 1-3, wherein the biopolymer is configured to support a cellular monolayer on a surface distal to the synthetic support membrane.

5. The reinforced biopolymer of any one of claims 1-4, wherein a layer of the biopolymer occurs on each side of the support membrane and the support membrane is approximately centrally located between two layers of the biopolymer.

6. The reinforced biopolymer of claim 5, wherein a layer of the biopolymer on only one side of the support membrane is configured to support a cellular monolayer.

7. The reinforced biopolymer of any one of claims 1-6, wherein the biopolymer is imbibed into the support membrane.

8. The reinforced biopolymer of any one of claims 1-7, wherein the synthetic support membrane (102) is biologically inert.

9. The reinforced biopolymer of any one of claims 1-8, wherein the synthetic support membrane (102) is a biaxially oriented ePTFE membrane having a crystallinity index of at least 94% and a matrix tensile strength in both the longitudinal and transverse directions of at least 600 MPa, wherein the crystallinity index and the matrix tensile strength are measured by the methods defined in the description.

10. The reinforced biopolymer of any one of claims 1-9, wherein the synthetic support membrane (102) is configured to sustain mass transfer across the thickness of the synthetic support membrane (102).

11. The reinforced biopolymer of any one of claims 1-10, wherein the reinforced biopolymer (100, 200, 300) has a thickness of 0.5 $\mu$m to 100 $\mu$m.

12. An implantable cell therapy device comprising the reinforced biopolymer (100, 200, 300) of any one of claims 1-11 and a monolayer of cells on a surface of the biopolymer (104) distal to the support membrane (102).

13. The implantable cell therapy device of claim 12, wherein the monolayer of cells includes cells selected from the group consisting of corneal endothelial cells, corneal epithelial cells, retinal pigment epithelial cells, photoreceptor cells, Müller glial cells, ganglion cells, endothelial cells, epithelial cells, pericytes, and combinations thereof.

**Patentansprüche**

1. Verstärktes Biopolymer (100, 200, 300), umfassend i) eine synthetische Stützmembran (102), beinhaltend zumindest eines von: expandiertem Polytetrafluorethylen (ePTFE); und expandiertem Polyethylen, und ii) ein Biopolymer (104), wobei das verstärkte Biopolymer Folgendes aufweist

   a. eine gemessene optische Transparenz von zumindest 85 %;
   b. eine Dicke von 100 $\mu$m oder weniger; und
   c. eine Zähigkeit von zumindest 30 KJ/m$^3$,
   wobei die synthetische Stützmembran eine Dicke von 0,5 $\mu$m bis 10 $\mu$m aufweist; und wobei eine einzelne Biopolymerschicht, die auf einer Seite der synthetischen Stützmembran auftritt, eine Dicke von 0 $\mu$m bis 95 $\mu$m aufweist,
   wobei die optische Transparenz, die Dicke und die Zähigkeit durch die Verfahren gemessen werden, die in der Beschreibung definiert sind.

2. Verstärktes Biopolymer nach Anspruch 1, wobei das Biopolymer ein Material ausgewählt aus der Gruppe bestehend aus Kollagen, Gelatine, Laminin, Fibronektin, Fibrinogen, Elastin, Thrombospondin, Heparansulfat, Chondroitinsulfat, Polysacchariden, Alginat, Chitosan, Glycosaminoglycan, Hyaluronsäure und Kombinationen davon beinhaltet.

3. Verstärktes Biopolymer nach Anspruch 1 oder Anspruch 2, wobei das Biopolymer funktionalisiert ist, um Zellwachstum, Zelladhäsion oder sowohl Zellwachstum als auch Zelladhäsion zu stützen.

4. Verstärktes Biopolymer nach einem der Ansprüche 1-3, wobei das Biopolymer dazu konfiguriert ist, eine zelluläre Monoschicht auf einer Oberfläche distal zu der synthetischen Stützmembran zu stützen.

5. Verstärktes Biopolymer nach einem der Ansprüche 1-4, wobei eine Schicht des Biopolymers auf jeder Seite der Stützmembran auftritt und sich die Stützmembran ungefähr mittig zwischen zwei Schichten des Biopolymers befindet.

**6.** Verstärktes Biopolymer nach Anspruch 5, wobei eine Schicht des Biopolymers auf nur einer Seite der Stützmembran dazu konfiguriert ist, eine zelluläre Monoschicht zu stützen.

**7.** Verstärktes Biopolymer nach einem der Ansprüche 1-6, wobei das Biopolymer in die Stützmembran aufgenommen ist.

**8.** Verstärktes Biopolymer nach einem der Ansprüche 1-7, wobei die synthetische Stützmembran (102) biologisch inert ist.

**9.** Verstärktes Biopolymer nach einem der Ansprüche 1-8, wobei die synthetische Stützmembran (102) eine biaxial orientierte ePTFE-Membran mit einem Kristallinitätsindex von zumindest 94 % und einer Matrixzugfestigkeit sowohl in der Längs- als auch Querrichtung von zumindest 600 MPa ist, wobei der Kristallinitätsindex und die Matrixzugfestigkeit durch die Verfahren gemessen werden, die in der Beschreibung definiert sind.

**10.** Verstärktes Biopolymer nach einem der Ansprüche 1-9, wobei die synthetische Stützmembran (102) dazu konfiguriert ist, Massentransfer über die Dicke der synthetischen Stützmembran (102) aufrechtzuerhalten.

**11.** Verstärktes Biopolymer nach einem der Ansprüche 1-10, wobei das verstärkte Biopolymer (100, 200, 300) eine Dicke von 0,5 $\mu$m bis 100 $\mu$m aufweist.

**12.** Implantierbare Zelltherapievorrichtung, umfassend das verstärkte Biopolymer (100, 200, 300) nach einem der Ansprüche 1-11 und eine Monoschicht aus Zellen auf einer Oberfläche des Biopolymers (104) distal zu der Stützmembran (102).

**13.** Implantierbare Zelltherapievorrichtung nach Anspruch 12, wobei die Monoschicht aus Zellen Zellen ausgewählt aus der Gruppe bestehend aus Hornhautendothelzellen, Hornhautepithelzellen, retinalen Pigmentepithelzellen, Photorezeptorzellen, Müller-Gliazellen, Ganglienzellen, Endothelzellen, Epithelzellen, Perizyten und Kombinationen davon beinhaltet.

**Revendications**

**1.** Biopolymère renforcé (100, 200, 300) comprenant i) une membrane support synthétique (102) comprenant au moins l'un des composés suivants : le polytétrafluoroéthylène expansé (PTFEe) ; et le polyéthylène expansé et ii) un biopolymère (104), le biopolymère renforcé présentant

   a. une transparence optique mesurée supérieure ou égale à 85 % ;
   b. une épaisseur inférieure ou égale à 100 $\mu$m ; et
   c. une ténacité supérieure ou égale à 30 KJ/m$^3$,

ladite membrane support synthétique présentant une épaisseur de 0,5 $\mu$m à 10 $\mu$m ; et une seule couche de biopolymère se trouvant sur un côté de la membrane support synthétique présentant une épaisseur de 0 $\mu$m à 95 $\mu$m, ladite transparence optique, ladite épaisseur et ladite ténacité étant mesurées par les méthodes définies dans la description.

**2.** Biopolymère renforcé selon la revendication 1, ledit biopolymère comprenant un matériau choisi dans le groupe constitué par le collagène, la gélatine, la laminine, la fibronectine, le fibrinogène, l'élastine, la thrombospondine, le sulfate d'héparane, le sulfate de chondroïtine, les polysaccharides, l'alginate, le chitosane, le glycosaminoglycane, l'acide hyaluronique et leurs combinaisons.

**3.** Biopolymère renforcé selon la revendication 1 ou la revendication 2, ledit biopolymère étant fonctionnalisé pour supporter la croissance cellulaire, l'adhérence cellulaire, ou à la fois la croissance et l'adhérence cellulaires.

**4.** Biopolymère renforcé selon l'une quelconque des revendications 1 à 3, ledit biopolymère étant conçu pour supporter une monocouche cellulaire sur une surface distale par rapport à la membrane support synthétique.

**5.** Biopolymère renforcé selon l'une quelconque des revendications 1 à 4, une couche du biopolymère se trouvant de chaque côté de la membrane support et ladite membrane support étant approximativement située au centre entre deux couches du biopolymère.

6. Biopolymère renforcé selon la revendication 5, une couche du biopolymère sur un seul côté de la membrane support étant conçue pour supporter une monocouche cellulaire.

7. Biopolymère renforcé selon l'une quelconque des revendications 1 à 6, ledit biopolymère étant imbibé dans la membrane support.

8. Biopolymère renforcé selon l'une quelconque des revendications 1 à 7, ladite membrane support synthétique (102) étant biologiquement inerte.

9. Biopolymère renforcé selon l'une quelconque des revendications 1 à 8, ladite membrane support synthétique (102) étant une membrane en PTFEe orientée biaxialement présentant un indice de cristallinité supérieur ou égal à 94 % et une résistance à la traction de matrice à la fois dans les directions longitudinale et transversale supérieure ou égale à 600 MPa, ledit indice de cristallinité et ladite résistance à la traction de matrice étant mesurés par les méthodes définies dans la description.

10. Biopolymère renforcé selon l'une quelconque des revendications 1 à 9, ladite membrane support synthétique (102) étant conçue pour supporter un transfert de masse à travers l'épaisseur de ladite membrane support synthétique (102).

11. Biopolymère renforcé selon l'une quelconque des revendications 1 à 10, ledit biopolymère renforcé (100, 200, 300) présentant une épaisseur de 0,5 $\mu$m à 100 $\mu$m.

12. Dispositif de thérapie cellulaire implantable comprenant le biopolymère renforcé (100, 200, 300) selon l'une quelconque des revendications 1 à 11 et une monocouche de cellules sur une surface du biopolymère (104) distale par rapport à la membrane support (102).

13. Dispositif de thérapie cellulaire implantable selon la revendication 12, ladite monocouche de cellules comprenant des cellules choisies dans le groupe constitué par les cellules endothéliales cornéennes, les cellules épithéliales cornéennes, les cellules épithéliales de pigment rétinien, les cellules photoréceptrices, les cellules gliales de Müller, les cellules ganglionnaires, les cellules endothéliales, les cellules épithéliales, les péricytes et leurs combinaisons.

100

104

102

104

FIG. 1

200

104

102

106

FIG. 2

300

104

102

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013040559 A1
- US 3953566 A, Gore
- US 7306729 B, Gore
- US 20120065649 A
- US 5814405 A, Gore

**Non-patent literature cited in the description**

- **KUIJPERS et al.** *Journal of Biomaterials Science, Polymer Edition*, 2000, vol. 11 (3), 225-243